(19) Europäisches Patentamt
European Patent Office
Office européen des brevets

(11) Publication number: 0 103 910 A1

(12) EUROPEAN PATENT APPLICATION

(21) Application number: 83200946.8

(22) Date of filing: 24.06.83

(51) Int. Cl.$^3$: A 61 K 7/48
A 61 K 9/10

(30) Priority: 30.06.82 US 394041

(43) Date of publication of application:
28.03.84 Bulletin 84/13

(84) Designated Contracting States:
AT BE CH DE FR GB IT LI NL SE

(71) Applicant: THE PROCTER & GAMBLE COMPANY
301 East Sixth Street
Cincinnati Ohio 45202(US)

(72) Inventor: Howard, Norman Bratton
4830 Stillwell Road
Oxford Ohio 45056(US)

(74) Representative: Suslic, Lydia et al,
Procter & Gamble European Technical Center Temselaan 100
B-1820 Strombeek-Bever(BE)

# (54) Emollient-containing skin conditioning compositions.

(57) Skin conditioning compositions are described which comprise an emulsion of petrolatum; water; an unsaturated monoglyceride emulsifier; and a skin conditioning agent, preferably glycerine. As a result of the novel cubic, viscous isotropic dispersed phase of these water-in-oil emulsions, they possess the ability to incorporate and deliver relatively high levels of glycerine while retaining high cosmetic acceptance.

EP 0 103 910 A1

0103910

# EMOLLIENT-CONTAINING SKIN CONDITIONING COMPOSITIONS

~~Norman B. Howard~~

## FIELD OF INVENTION

The present invention relates to compositions effective in making certain agents bioavailable by delivery of said agents through the integument as well as possessing the ability to act as a skin conditioner by delivery of emollients through the skin.

Because of the ease of access, dynamics of application, vast exposure to the circulatory and lymphatic networks, large surface area, and noninvasive nature, the delivery of agents through the skin has long been a promising route. This is true whether the bioavailability desired is systemic or dermal, regional or local.

The advantages of this form of delivery include, but are not limited to: avoidance of risks associated with parenteral treatment; elimination of the inconveniences of parenteral treatment; avoidance of the variable rates of absorption and metabolism inherent in oral treatment; increasing the continuity of drug administration by permitting delivery of agents with short biological half-lives; and elimination of gastrointestinal irritation resulting from exposing the gastrointestinal tract to pharmaceutical actives, preservatives, tableting agents, and the like. Most importantly, topical delivery possesses the ability to treat local manifestations of diseases or other abnormal conditions both systemically and locally. The use of such topical formulations as skin conditions to deliver emollients through the skin as, for example, the treatment of dry skin, is widely-used and well-accepted.

However, because it must serve as a barrier to the ingress of pathogens and toxic materials, and the egress of physiologic fluids, the skin is highly impermeable. It must be impermeable to both lipid soluble materials to preserve its own integrity, and to water soluble materials to preserve the delicate electrolyte balance of the body.

A good deal of this impermeability results from the nature of one very thin layer created by normal developmental

and physiologic changes in the skin. As cells are formed in the basal layer, they begin to migrate toward the surface, until they are eventually sloughed off. As they undergo this migration, they become progressively more keratinized. When they reach the surface, just prior to being discarded, they form a thin layer approximately 10 microns thick. This layer is called the stratum corneum. As a result of this high degree of keratinization of the cells which comprise the stratum corneum, it poses a formidable barrier. Therefore, penetration via the non-polar route (across the cell) remains most difficult.

Other possible penetration routes are available. First, any mechanism which allows the egress of materials, e.g. the sebaceous apparatus, can be manipulated to allow the ingress of materials. Second, the stratum corneum, though keratinized to a great degree, is composed of about 15% lipid-based intercellular material. This material may offer a less formidable route despite the close packing of the cells.

Because of the three very different routes, each with its own distinct mechanism, it is difficult to find one composition to act as a vehicle which effectively delivers lipid soluble agents, water soluble agents, or both simultaneously, and which can do so in such a manner so as to make the agent bioavailable systemically as well as locally.

The present invention relates to the discovery that certain water-in-oil emulsions, when the external phase exists as a viscous isotropic dispersed phase, can effectively deliver both lipid soluble and water soluble agents, alternatively or simultaneously. In particular, it has been discovered that certain unsaturated monoglycerides will yield this novel, stable viscous isotropic dispersed phase when used to emulsify a water-in-oil or (water-in-oil)-in water emulsion of water and petrolatum. See copending United States Patent Application Serial Number ______, Emollient-Containing Topical Pharmaceutical Compositions, Hong, et al., filed __________, expressly incorporated herein by reference.

The enhanced penetration provided by the present invention now makes the topical mode of treatment practical by

percutaneously delivering clinically effective doses of lipid soluble or water soluble agents in a form that makes them available both systemically and locally.

Once a composition which is effective in opening a pathway for effective penetration of the skin is prepared and applied, a second significant problem is immediately perceived. Such compositions are frequently irritating. It will quickly be appreciated that this irritation should be expected. When a pathway for ingress is opened, egress of physiologic compounds and complexes also occurs, due to the integument having been compromised. Thus an acceptable vehicle must not only effectively deliver lipid soluble and/or aqueous soluble agents, both systemically and locally, but also provide a mechanism to prevent egress of physiologic material. This mechanism is provided by the present invention in a manner which does not interfere with the ability to make the active bioavailable.

This impermeability of the stratum corneum also makes it difficult to condition the skin. It is the stratum corneum which governs the diffusion of water in and out of the skin. And though it does not give up water easily, it is well-known that the skin has a tendency to dry out when exposed to conditions of low humidity, or other harsh conditions, for extended periods of time. This loss of water causes the skin to lose its smooth and supple natural appearance and feel.

Because of its barrier nature, the stratum corneum prevents simple rehydration once it has allowed water loss. Thus, to alleviate this dryness, a variety of compounds have been used to increase the water content of the stratum corneum itself, as well as the tissue below it. These compounds, generally called emollients and humectants, range from compounds which attempt to rehydrate the skin with water-miscible agents to those which lubricate/occlude the skin with water insoluble agents. It is well-known that glycerin is the best of the former, while petrolatum is the best of the latter.

While petrolatum is the most efficient occludent for protecting dry skin and aiding rehydration, it is also a substance

that is difficult to emulsify. Accordingly, it has the least amount of cosmetic acceptance, especially when emulsified to incorporate a polyol, such as glycerine, to aid in rehydration. See Sason, Cosmetic Technology, pp 49-50, (May 1982). Therefore, one is faced with this "cosmetic paradox" of skin conditioning performance and cosmetic acceptance running in opposite directions, i.e. less effective conditioners (low-viscosity mineral oil-in-water emulsions) having much greater cosmetic acceptance than highly effective ones (ultra high-viscosity petrolatum emulsions). This poses a formulation dilemma in that rehydration of the skin cannot be the sole object of a skin conditioning composition, the user's perception of the state of the skin is also critical.

The present invention, therefore, also relates to the discovery that the novel water-in-oil emulsions of the present invention provide a petrolatum + glycerine composition which is effective as a skin conditioner, and is also a gravitationally and thermally stable emulsion which has high cosmetic acceptance compared to other petrolatum preparations.

## BACKGROUND

Many fruitless, incomplete or inadequate attempts have been directed toward formulating an emulsion which possesses the advantages of the present invention.

Emollients are materials used for local application to prevent or relieve dryness, and to protect the skin.

Glycerine (1,2,3-propane triol; glycerol; glycerin) is a byproduct of the manufacture of soaps and fatty acids. It is known as a solvent, humectant, plasticizer and emollient. The Merck Index, 9th Ed., p. 581.

Water is an effective emollient, as well as the only known plasticizer of the stratum corneum.

Petrolatum is a colloidal system of nonstraight-chain solid hydrocarbons and high-boiling point liquid hydrocarbons held inside micelles. It has been known as a base for incorporation of glycerine since its discovery by Robert A. Chesebrough. See U.S. Patent 127,568, issued June 4, 1872 to Chesebrough.

U.S. Patent 3,536,816, issued October 27, 1970 to Kellner, discloses water-in-oil emulsion vehicles for cosmetics and pharmaceuticals containing sorbitol.

U.S. Patent 3,919,430, issued November 11, 1975 to Siegel, discloses water absorption bases useful in cosmetic and pharmaceutical formulations containing at least 75% petrolatum.

U.S. Patent3,957,969, issued May 18, 1976 to Fujiyama et al., discloses a cosmetic stick prepared from a water-in-oil emulsion. This stick is designed to possess properties to allow spreading on human skin.

U.S. Patent 4,216,201, issued August 5, 1980 to Calvo, discloses water-in-oil emulsion bases for cosmetic creams and lotions to enhance moisture penetration.

U.S. Patent 3,635,834, issued January 18, 1975 to Celento et al., discloses process steps for preparing homogenized semisolid emulsions of either the oil-in-water or water-in-oil type.

Sagarin, Cosmetics Science and Technology, 2nd Edition, Vol. 1, Wiley Interscience (1972), recommends numerous water-in-oil and oil-in-water emulsions in Chapter 2 and Chapter 3.

A trade publication entitled Food Emulsifiers, Coatings and Lubricants, published by Eastman Chemical Products, 1981, discloses high-melting distilled unsaturated monoglycerides as useful as emulsifiers and viscosity-adjusting additives in foods, cosmetics and pharmaceutical.

While the above disclose materials of the type present in the compositions of the invention described herein, they do not disclose the instant composition. The above also fail to suggest forming such compositions with the critical viscous isotropic phase or the benefits associated therewith.

It is an object of the present invention to provide effective topical products which are cosmetically and/or therapeutically acceptable.

It is also an object of the present invention to provide a composition which can incorporate relatively high levels of glycerine while retaining cosmetic acceptance.

It is a further object of the present invention to provide an improved method for conditioning skin.

It is still a further object of the present invention to provide compositions which allow the incorporation of petrolatum as an efficient occludent and/or emollient but which are cosmetically acceptable.

It is still a further object of the present invention to provide improved compositions and methods for topical delivery of pharmaceutical agents without irritation.

It is still a further object of the present invention to provide a composition which can make bioavailable clinically effective levels of pharmaceutical agents.

These and other objects will be readily apparent from the detailed description which follows.

All percentages used herein are by weight unless otherwise indicated.

## SUMMARY OF THE INVENTION

The compositions of the present invention comprise from about 9% to about 89% petrolatum; from about 10% to about 90% water; about 0.25% to about 10% of an emulsifier selected from glyceryl monooleate, glyceryl monolinoleate, or mixtures thereof; and about .01% to about 25% of a skin conditioning agent.

## DETAILED DESCRIPTION OF THE INVENTION

The essential components of the composition of the present invention, as indicated above, as well as optional components are specified below.

### Petrolatum

Petrolatum (petroleum jelly; paraffin jelly; vasoliment; Vaseline) is one of the essential components of the compositions described and claimed herein. Petrolatum is a purified mixture of semisolid hydrocarbons of the general formula $C_n H_{2n+2}$, where n = 16-32. Premium petrolatum is a white, semisolid, unctious mass which is odorless and tasteless. It is a product of commerce.

In the present compositions petrolatum is present at a level of from about 9% to about 89%, preferably about 10 to about

70%, and more preferably at a level of from about 10% to about 50%. It will be appreciated that the level of petrolatum will vary according to the pharmaceutical or skin conditioning agent selected, and how much of an occlusive barrier is needed to render a particular formulation efficacious.

## Water

Water is one of the essential components of the present compositions and is generally present at a level of about 10% to about 90%, preferably about 30 to about 80%, and more preferably present at a level of about 50% to about 80%.

## Emulsifier

Another essential component of the present composition is an emulsifier or mixture of emulsifiers which will be capable of emulsifying the water component in the petrolatum such that a thermally and gravitationally stable viscous isotropic phase is achieved at room temperature by the dispersed phase. Such emulsifiers fall into a very limited class of unsaturated monoglycerides.

An example of such an emulsifier is glyceryl monooleate, which is the monoester of glycerine and oleic acid. It is generally represented by the formula

$$(CH_3)(CH_2)_7\ CH = CH(CH_2)_7\overset{\overset{O}{\|}}{C}\text{-}OCH_2CH(OH)CH_2OH$$

Another example is glyceryl monolinoleate, which is the monoester of glycerine and linoleic acid. It is generally represented by the formula

$$(CH_3)(CH_2)_4\ CH{=}CHCH_2\ CH{=}CH(CH_2)_7\ \overset{\overset{O}{\|}}{C}\text{-}OCH_2\ CH(OH)CH_2OH$$

Mixtures of these two monoesters will also work in the present invention.

The emulsifier is present at a level of about 0.25% to about 10%, more preferably about 0.5 to about 5%, and most preferably about 1% to about 3%, of the total composition by weight.

Pharmaceutical Agents and Skin Conditioning Agents

The compositions of the present invention must include, in addition to the aforementioned essential components, one or more of a wide variety of oil-soluble, water-soluble, or oil- and water-soluble pharmaceutical agents or skin conditioning agents.

Skin Conditioning Agents

Among the water-soluble skin conditioning agents preferred in the present invention is glycerine. Glycerine is 1,2,3-propanetriol and is a product of commerce. One large source of the material is the manufacture of soap.

In the present compositions glycerine is present at a level of about 0.5% to about 25%, preferably at a level of from about 5% to about 20%. Levels outside of these ranges either provide inadequate skin conditioning, or provide no better skin conditioning than can be achieved with levels within these ranges. Levels outside these ranges also provide compositions exhibiting unacceptable aesthetic qualities.

In general, many other skin conditioning agents may be used in the present invention. They may be either lipid-soluble or water-soluble. Lipophilic skin conditioning agents may be incorporated into the petrolatum phase of the present invention, whereas hydrophilic skin conditioning agents may be incorporated into the aqueous phase of the present invention.

The categories of skin conditioning agents useful in the present invention include, but are not limited to emollients, humectants and plasticizers.

Humectants other than glycerine may be present in the compositions of this invention. These include, but are not limited to: sorbitol; propylene glycol; alkoxylated glucose; hexanetriol; ethanol; and the like. Emollient materials other than glycerine may be used in the present invention. These include, but are not limited to, hydrocarbon oils and waxes; silicone oils; triglyceride esters, acetoglyceride esters, ethoxylated glycerides; alkyl esters; alkenyl esters; fatty acids, fatty alcohols; fatty alcohol ethers; ether-esters (fatty acid esters of ethoxylated fatty alcohols); lanolin and its derivitives; polyhydric alcohols and

polyether derivatives; polyhydric alcohol esters; wax esters; beeswax derivatives; vegetable waxes; phospholipids; steroids; and amides. These may all be used at art-established levels. These materials are all described in detail in Sagarin, Cosmetics Science and Technology, 2nd Edition, Wiley-Interscience (1972), Vol. 1, pp. 27-104 and pp. 179-222, incorporated herein by reference.

Pharmaceutical Agents

Among the lipid soluble pharmaceutical agents preferred in the present invention is indomethacin. Indomethacin; (1-(p-Chlorobenzoyl)-5-methoxy-2-methylindole-3-acetic acid; 1-(p-chlorobenzoyl)-5-methoxy-2 methyl-3-indolylacetic acid; Indocin),

(I)

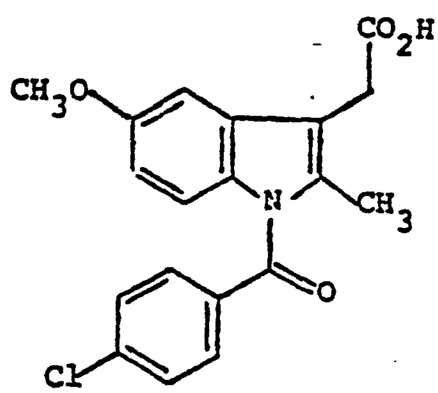

represented by (I), is an effective nonsteroidal anti-inflammatory agent whose use and preparation are well-known. See Scherrer and Whitehouse, Anti-inflammatory Agents, Chemistry and Pharmacology, Vol. 1, Academic Press (1974), pp 91-127, 245-294, incorporated herein by reference.

In the present composition indomethacin is present at a level of from about .01% to about 25%, and preferably about 0.5% to about 5%. Indomethacin is most preferably present at levels of about 1% to about 3%.

In general, many other pharmaceutical agents may be used in the present invention. They may be either lipid-soluble

or water-soluble. Lipophilic pharmaceutical agents may be incorporated into the petrolatum phase of the present invention, whereas hydrophilic pharmaceutical agents may be incorporated into the aqueous phase of the present invention.

The categories of pharmaceutical agents useful in the present invention include, but are not limited to: nonsteroidal anti-inflammatory agents, local anesthetics, antibiotics, steroids, benzoyl peroxide, and antimicrobials. Other penetration aids or vehicles may also be used.

As stated above, many pharmaceutical agents may be used in addition to or in place of those listed above. Nonsteroidal anti-inflammatory agents other than indomethacin are useful in the present compositions. These include, but are not limited to, salicylic acid, ibuprofen, sulindac, naproxen, ketoprofen, etofenamate, and their pharmaceutically acceptable salts and esters. These are described in detail in Scherrer and Whitehouse, Anti-inflammatory Agents, Chemistry and Pharmacy, pp 91-127, previously incorporated by reference. Goodman and Gilman, The Pharmacological Basis of Therapeutics, 5th Edition, MacMillan Publishing Co. (1975), pp 325-358, incorporated herein by reference, also contains much information on the effective use of these nonsteroidal anti-inflammatory agents. See also Great Britain Patent 1,588,284, incorporated herein by reference. Preparation of these compounds is well within the skill of the art, in addition to being thoroughly treated in the above references. The penetration of the above agents is enhanced by the present compositions.

Another group of pharmaceutical agents which can be used in the present compositions with enhanced delivery are the local anesthetics. These include, but are not limited to lidocaine, procaine, mepivacaine, bupivacaine, dibucaine, and tetracaine, as well as their pharmaceutically-acceptable salts and esters.

Local anesthetics may be incorporated at art-established levels. The use and preparation of such agents is well within the skill of the artisan.

It will be appreciated that selection of a local anesthetic requires the evaluation of several factors. A preferred local anesthetic should not be irritating to the tissue, or at least the level of irritation should not exceed that of the vehicle or other actives; it should not cause any permanent nerve damage; its systemic toxicity after absorption should be low; the time required for the onset of anesthesia should be as short as possible; and the duration should be appropriate for the procedure to be conducted or the condition to be treated.

Among the local anesthetics, few have the safety and efficacy record accumulated by lidocaine. Thus, lidocaine is a preferred pharmaceutical agent for use as a local anesthetic in the compositions of the present invention. Lidocaine (lidocaine hydrocloride) may be present at a level of about .05% to about 5%, preferably about 1% to about 4%, and most preferably at a level of about 1% to about 3%.

Another group of pharmaceutical agents which are especially suitable for topical administration and can be used in the present invention with enhanced delivery are the antibiotics, a subgroup of antimicrobials. These include, but are not limited to, erythromycin, penicillin, clindamycin, tetracycline, and chloramphenicol, as well as their pharmaceutically-acceptable salts and esters.

Antibiotics may be incorporated at art-established levels. The use and preparation of such agents is well within the skill of the artisan.

For the antibiotic treatment of acne, erythromycin, clindamycin and tetracycline are especially useful and preferred for use in the composition of this invention. Another combination especially useful and preferred which is suitable for the treatment of acne is a combination polymyxin B, bacitracin, and neomycin.

When selecting an antibiotic to treat non-acne dermatologic infections, penicillin is almost the universal drug of first choice. Accordingly, penicillin, synthetic penicillins, particularly penicillinase-resistant varieties, also are especially preferred.

Another agent which is especially preferred for systemic use, particularly in patients with sensitivity to the penicillins, is erythromycin, including its pharmaceutically-acceptable salts and esters. Preferred examples include erythromycin ethylsuccinate, erythromycin lactobionate, and erythromycin estolate. A third especially preferred antibiotic embodiment is a combination for use within the present composition: polymyxin B, bacitracin and neomycin.

Another group of pharmaceutical agents which are suitable for use within the compositions of the present invention, for both delivery as a pharmaceutical active and to preserve the composition itself, are antimicrobials. By antimicrobial, as used herein, is meant any agent, endogenous or synthetic, that will suppress the growth of microorganisms, and may eventually destroy them.

The selection of an appropriate antimicrobial is well within the skill of the art. It will be appreciated that the major factors in selecting an antimicrobial, (once such therapy is indicated) will be the etiology of the infectious process, the sensitivity pattern of the infecting agent, treatment resistence of the microbe, the cost of the agent, and the efficacy record of this agent in the subject, when available.

$C_5$-$C_{12}$ fatty acids are antimicrobial agents particularly useful within the present invention. The materials, themselves, are well-known in the art. For example, octanoic acid is an oily liquid having a boiling point of 239.7°C and a melting point of 16.7°C; it may be prepared from 1-heptene, Dupont, et al., Compt. Rend. 240, 628 (1955), or by the oxidation of octanol, Langenbeck, et al., Ber., 89, 202 (1956).

$C_5$-$C_{12}$ fatty acids and fatty acid salts are known to be effective antimicrobial and antifungal agents. Keeney, Bull. Johns Hopkins Hosp. 78, 333 (1946), teaches the use of a 20% aqueous solution of sodium caprylate, at pH 7.4, to successfully treat moniliasis. U.S. Patent 2,466,663, Russ et al., issued April 5, 1949, discloses compositions having pH's between 4.5 and 10.5, containing a mixture of caprylic acid and a caprylic acid

salt, especially zinc or sodium caprylate. These solutions are taught to be useful in preventing the growth of molds or fungi in foods and other nutrient media. Theodore, JAMA 143, 226(1950), discloses that the lower fatty acids have been shown to be of value in the treatment of external infections of the eyes. Specifically, sodium propionate was found effective against bacteria causing common occular and fungal infections, including staphylococcus and *Pseudomonas aeruginosa*. Copending U.S. Patent Application Serial No. 918,532, Stone, filed June 23, 1978, describes the use of $C_4$-$C_9$ fatty acids, especially octanoic acid, as broad spectrum antimicrobial agents in intravenous, nutrient and dialysis solutions; these solutions typically contain electrolytes, such as sodium chloride. The antimicrobial benefits of maintaining a solution pH between about 3.5 and 6.0 are taught.

It is preferred that the fatty acid component used in the present invention be a $C_5$-$C_{12}$ non-aromatic carboxylic acid, such as n-pentanoic, n-hexanoic, n-heptanoic, n-octanoic, n-nonanoic, n-decanoic, n-undecanoic, or n-dodecanoic acid. Preferred fatty acid materials for use in the present invention contain from 6 to 10 carbon atoms. Mixtures of the above fatty acids as well as their salts may be used, provided that the pH criteria for the compositions, as described below, are met.

The compositions of the present invention contain from about 0.01% to about 10% of the selected antimicrobial. The compositions are formulated to have a pH of no greater than about 6.5 and no less than about 3.5; preferably the pH falls between about 5 and about 3.5. At pH's above this range, the antimicrobial performance of the composition falls off significantly. Compatible acidic, basic or buffering ingredients may be used to adjust the pH to the desired range, and to maintain it at the preferred range when applied.

It will be appreciated that the level of antimicrobial will depend upon many factors. Levels incorporated for penetration enhancement will generally be higher than levels incorporated to preserve the composition itself.

Another group of pharmaceutical agents which can be used in the present compositions with enhanced delivery are the anti-inflammatory steroids. By "anti-inflammatory steroid", as used herein, is meant steroids which have an anti-inflammatory activity either locally or systemically. These are well-known within the art. See Scherrer and Whitehouse, Anti-inflammatory Agents, Chemistry and Pharmacology, pp 245-294, previously incorporated by reference. Preferred steroids are the adrenocorticoid steroids, whether endogenous or synthetic. These include, without limitation, hydrocortisone, betamethasone, cortisone, dexamethasone, prednisolone, prednisone, methylprednisilone, triamcinolone, flumethasone, and their pharmaceutically-acceptable derivitives. Most highly preferred among these are hydrocortisone and cortisone.

Steroid selection and preparation is well within the skill of the artisan, and they may be used at art levels. Steroids are preferred in the present compositions at levels of from about 0.01 to about 2.5%, and levels of about 0.025 to about 0.25 are highly preferred.

Another pharmaceutical agent which can be used in the present compositions with enhanced delivery is benzoyl peroxide, an item of commerce. Such compositions are useful in the treatment of acne. It is preferably present at levels of from about 0.5% to about 25%, and more preferably from about 2% to about 10%. Though levels higher than 10% are equally or more effective, the skin irritation which commonly accompanies the topical use of benzoyl peroxide is generally the limiting factor. It will be appreciated that benzoyl peroxide is a relatively powerful oxidizing agent, and therefore when used with the unsaturated emulsifiers of this invention care must be used in formulation. Therefore, it is suggested that an antioxidizing agent be used to protect the stability of the viscous isotropic phase.

The compositions of the present invention may also be used in concert with other penetration vehicles or aides. For example, copending U.S. Patent Application Serial No. 296,706, Cooper et al., filed August 25, 1981, describes the use of a

system comprising a polar diol solvent and a "cell envelope-disordering" compound as a penetration enhancing vehicle. See European Patent Application 43,738, incorporated herein by reference. Such a vehicle may be used simultaneously with this invention, or use of this invention may proceed or follow the use of the other vehicles to further enhance penetration and/or reduce irritation. In a preferred embodiment of this invention, the compositions of instant invention are combined with the compositions of Copending U.S. Patent Application Serial No. 296,706, described above. Penetration is enhanced and irritation is reduced.

The compositions of the present invention may likewise be used as described above with the penetrating compositions antiviral actives, or both, of U.S. Patent Application Serial Number 383,891, Penetrating Topical Pharmaceutical Compositions Containing 9-(2-hydroxyethoxymethyl) guanine, Cooper, filed June 1, 1982.

The compositions of the present invention, when combined with the penetrating compositions above, can be either physically blended, or, the cell-envelope-disordering compounds may be dissolved in the appropriate phase prior to preparing the final emulsion as described below.

Further, the compositions of the present invention may be used with other skin conditioning compositions which contain glycerine. They may be used consecutively, conjointly or as a single composition. When used as a single composition, the viscous isotropic water-in-oil emulsions of the present invention are incorporated into a second water-in-oil or oil-in-water emulsion useful as a skin conditioner, preferably one which also contains glycerine. They can be incorporated at a ratio of about 99:1 to about 1:99 second emulsion:emulsion of the present invention. This ratio for incorporation is preferably about 20:1 to about 1:20, and most preferably about 10:1 to about 1:1. For example, in a preferred embodiment of the present invention, the compositions of the present invention are used in combination with the skin conditioning compositions of copending U.S. Patent

Application Serial Number 306,807, Dixon et al., filed September 29, 1982, incorporated herein by reference, at a ratio of about 20:1 to about 1:20. The two emulsions are combined by gently blending or folding them together. If this blending or folding is too violent, or if too much shear is used, the separate integrity of the emulsions will be lost, and accordingly, the respective benefits.

When combined with such compositions, in the ratio above, the overall ranges of certain required components will change. Accordingly, when combined with a second emollient-containing skin composition, the petrolatum may be present at a level of about 1% to about 89% of the final composition. The glycerine may be present at a level of about 5% to about 24% of the final composition, and the unsaturated monoglyceride emulsifier is present at a level of about 0.025% to about 10% of the final composition.

Other optional components can be added to the formulations of this invention to enhance their cosmetic acceptability. Such components include thickening agents, such as methylcellulose, cross-linked carboxypolymethylene polymers, bentonite, gum tragacanth, gum karaya, and the like. Thickeners are generally employed at a level of about 1% to about 10% of the total compositions.

Minor amounts of other materials may be added at art-established levels. These include, without limitation, pigments, opacifiers, fragrances, perfumes, and the like. Such materials, when added, should not unduly interfere with the penetration enhancement, or the conditioning efficacy, of these compositions. Such formula modifications to improve cosmetic acceptability are well within the skill of the workers in the cosmetic and dermatological arts and, by themselves, constitute no part of this invention.

It can be seen from the foregoing that the compositions admit of considerable variation, so long as the critical viscous isotropic dispersed phase of the water-in-oil petrolatum + water +

emulsifier + agent emulsior is present, using the ranges indicated above.

METHODS OF USE

It can be readily appreciated that this invention provides a method of percutaneous delivery of pharmaceutical agents, comprising topically applying to a human or lower animal in need of treatment with said pharmaceutical agent a safe and effective amount of a composition according to this invention containing a safe and effective amount of said pharmaceutical agent.

It will also be readily appreciated that this invention provides a method for prevention or relief of dryness, as well as protection of the skin, comprising applying topically to a human or lower animal in need of such treatment a safe and effective amount of a composition according to this invention containing a safe and effective amount of glycerine.

This invention additionally provides a method for treating and preventing pain in a human or lower animal, comprising applying topically to a human or lower animal in need of such treatment a safe and effective amount of at least one of the aforementioned local anesthetics.

This invention also provides a method for treating and preventing microbial infections in human and lower animals, comprising applying topically to a human or lower animal in need of such treatment a safe and effective amount of the composition of the present invention containing a safe and effective amount of at least one of the aforementioned antimicrobial agents.

This invention also provides a method for treating and preventing bacterial infection in human and lower animals, comprising applying topically to a human or lower animal in need of such treatment, a safe and effective amount of the composition of this invention containing at least one of the aforementioned antibiotic agents.

This invention also provides a method for treating pain and inflammation in humans and lower animals, comprising applying topically to a human or lower animal in need of such treatment a safe and effective amount of the composition according to this

invention containing a safe and effective amount of at least one of the nonsteroidal anti-inflammatory agents.

This invention also provides a method for treating and preventing pain and inflammation in human and lower animals, comprising applying topically to a human or lower animal in need of such treatment a safe and effective amount of the composition according to this invention, containing a safe and effective amount of at least one of the steroidal anti-inflammatory agents.

Finally, this invention also provides a method for treating and preventing acne, comprising applying to the affected situs a safe and effective amount of a benzoyl peroxide composition as provided herein. It will also be apparent that the present invention provides compositions and methods ideally suited for the antibiotic treatment of acne.

The compositions of this invention are typically applied twice daily to the affected situs, or, when a systemic effect is desired, to a larger area, depending upon the dose desired. A typical safe and effective usage rate is about 1 mg of the total composition per square centimeter of skin to about 50 mg of the total composition to a square centimeter of skin per application, but this can vary with the use, severity of the affliction, the physical and medical condition of the patient, other treatment concurrently administered, and the nature and concentration of the pharmaceutical agent, as well as the particular composition of the topical carrier being used. In particular, substantially higher application rates (up to 1,000 mg per square centimeter of skin) can be employed when a high degree of occlusive barrier is desired. It must be noted that application rates of the total composition, not just the drugs, are critical.

The compositions can be applied qd, q12h, q8h, q6h, q4h, or in any other convenient treatment regiment. A q4h schedule is particularly preferred because it minimizes the amount of drug which is applied at one time, without requiring inordinately frequent applications or amounts of the composition.

When the present composition is used with a safe and effective amount of a local anesthetic it can be applied on an "as-needed" basis, e.g. PRN.

When the present compositions are used to prevent or relieve dryness or to generally condition the skin they may be applied whenever the skin is perceived to be dehydrated. They may also be used once a day to about 6 times a day to prevent dehydration.

By "topical administration" as used herein, is meant directly laying on or spreading on the dermal tissue, especially outer skin.

By "safe and effective amount" of the compositions, or pharmaceutical agent, as used herein, is meant a sufficient amount of the composition or pharmaceutical agent to alleviate or prevent the abnormal or diseased state being treated within a reasonable benefit/risk ratio attendant with any medical treatment. Within the scope of sound medical judgment, the amount of the composition or pharmaceutical agent will usually vary with the particular condition being treated, the severity of the condition, the duration of the treatment, the specific compound or pharmaceutical agent employed and its concentration, and like factors within specific knowledge and expertise of the patient or the attending physician.

By "toxicologically- or pharmaceutically-acceptable" as used herein, is meant ingredients which are suitable for use in contact with the tissues of human or lower animals without undue toxicity, irritation, allergic response, and the like, commensurate with the reasonable benefit/risk ratio.

By the term "comprising" as used herein, is meant that the various and other compatible drugs and medicaments, as well as ingredients in cosmetic vehicles, can be conjointly employed in the compositions or processes of this invention, as long as the critical penetration enhancing viscous isotropic phase is achieved.

By "afflicted situs" or "affected situs", as used herein, is meant a localized area of inflammation or lesion, dryness, or the immediate surrounding area.

By "acne" as used herein, is meant common acne, acne vulgaris, and all forms, including papular, pustular, or cystic.

By "nonsteroidal anti-inflammatory agent", as used herein, is meant, generally, those drugs which appear to act, at least in part, by inhibition of prostaglandin synthetase. The variety of compounds encompassed by this phrase is well-known to those skilled in the art, and reference may be had to standard text, including Scherrer and Whitehouse, Anti-inflammatory Agents, Chemistry and Pharmacology, pp 91-127, previously incorporated herein by reference. This will provide detailed disclosures of chemical structures, syntheses, side-effects, and the like.

By "penetration enhancing" or "enhanced delivery" as used herein is meant that the viscous isotropic "penetrating enhancing" compositions of this invention provide marked transepidermal delivery of an incorporated pharmaceutical agent, when compared to other compositions at equal chemical potential. This latter aspect is important, since varying solubility of drugs in the various comparable vehicles will necessarily effect their transporting across the skin. Thus, for example, if a drug is soluble in vehicle A to the extent of 24%, and in vehicle B to the extent of 4%, if the compositions were to be compared at equal percentage concentrations, rather than equal chemical potential, the lower solubility composition will show a misleadingly 6-fold difference in transport over the more soluble vehicle. The simplest way of assuring equal chemical potential for evaluating penetration enhancement or enhanced delivery is to use saturated solutions or solutions of equal percentage of saturation of pharmacological active or pharmaceutical agent in the various vehicles.

By "local anesthetic" as used herein is meant drugs that block nerve conduction when applied locally to nerve tissue in appropriate concentrations. Their action is reversible, their use being followed by complete recovery in nerve function with no evidence of structural damage to the nerve fibers or cells.

By "antimicrobial agent" as used herein, is meant an agent which has the capacity to inhibit the growth of other microorganisms or destroy them.

By "antibiotic agent" as used herein, is meant a chemical substance producted by microorganisms or a derivative of a chemical substance produced by microorganisms that has the capacity to inhibit the growth of other microorganisms or destroy them.

By "steroidal anti-inflammatory agent" as used herein, is meant the variety of compounds well-known to one skilled in the art to be encompassed by this term, and reference may be had to standard texts, including Scherrer and Whitehouse, Anti-inflammatory Agents, Chemistry and Pharmacology, pp 245-294. This will provide detailed disclosure of chemical structures, synthesis, side-effects and the like.

By "viscous" as used herein, is meant that the viscosity of the dispersed phase composition is sufficiently large to make the viscous forces a significant part of the total force field in the composition.

By "isotropic" as used herein, is meant that the rheological or visual properties of the crystaline structure of the dispersed phase of the composition are not dependent upon the direction in which they are measured.

The critical viscous isotropic phase of the present composition is both thermally and gravitationally stable. It can be identified by noting a non-birefringent, viscous phase under a microscope using polarizing light. A second method to identify this structure can be performed in accordance with Laughlen's "An Expedient Technique for Determining Solubility Phase Boundaries in Surfactant-Water Systems," Journal of Colloid and Interface Science, 551, 239-241, (1976). It can also be identified using standard X-ray diffraction techniques for the identification of liquid crystals. X-ray powder techniques may also be employed if small angle regions are recorded as viscous isotropic cubic phases often have large repetition units, frequently up to several hundred Angstroms. See Friberg, Food Emulsions, Marcel

Dekker, Inc. NY (1976), pp 56-61, incorporated herein by reference. See also Abrahamsson, et al., Chem. Phys Lipids 8, 152 (1972); A. Tanicu, et al., Biochem Biophs Acta 109, 11 (1970). Finally, identification is possible by formation of the emulsion followed by centrifugation. When the water phase is separated out, it is found to be uniquely viscous, transparent and isotropic, when compared to any other water-in-oil petrolatum emulsion.

The compositions of the present invention may be prepared as follows. First, all lipid-soluble actives, agents and components selected for use are dissolved in the lipid phase (petrolatum). Next, all water-soluble actives, agents and components selected for use are dissolved in the aqueous phase (water). The lipid phase is heated to about 70°C. While at this temperature, the lipid phase is added together at a flow rate of about 1:1 under high shear to the ambient temperature aqueous phase. The high shear may be provided by any high shear type mixer known in the art, for example, a homogenizer, colloid mill, or the like. The amount of shear will depend upon the range and level of the necessary components, the optional components, as well as the eventual flow rate.

The following are nonlimiting examples of compositions and methods and use of the present invention.

The compositions of Examples I-VI are prepared by dissolving the lipid soluble ingredients in the petrolatum, and the water soluble ingredients in the water. The petrolatum is then heated to 70°C, and mixed in a zone of high shear, at a rate of 1:1, with the ambient temperature aqueous solution. When viewed under a microscope using a polarized light source, the compositions of Examples I-VI, as well as compositions identified as Part 1 A and B of Example VII, exhibit a non-birefringent viscous dispersed phase characteristic of the cubic, viscous isotropic phase of the present invention.

## EXAMPLE I

A composition of the present invention containing a dispersed, viscous isotropic phase is prepared as described above

using the following components.

| | Ingredient | Percent by Weight of Total Composition |
|---|---|---|
| 1. | Petrolatum | 49% |
| 2. | Glycerine | 10% |
| 3. | Glyceryl monooleate | 1% |
| 4. | Water | 40% |

This composition is topically applied to dry skin, as needed and rubbed in. It is applied at a rate of 5 milligrams of total composition per square centimeter of dry skin.

EXAMPLE II

| | Ingredient | Percent by Weight of Total Composition |
|---|---|---|
| 1. | Petrolatum | 49% |
| 2. | Indomethacin | 1% |
| 3. | Glyceryl monolinoleate | 1% |
| 4. | Water | 49% |

This composition is topically applied four times daily to a human suffering from arthritis. 10 milligrams of total composition is applied per square centimeter of skin of the desired application site.

In the foregoing composition, the indomethacin active can be replaced by other nonsteroidal anti-inflammatories, including, without limitation, salicylic acid, methyl salicylate, glycol salicylate, benzyl-2,5-diacetoxybenzoic acid, ibuprofen, sulindac, naproxen, ketoprofen, etofenamate, phenylbutazone, and other compounds of the formula

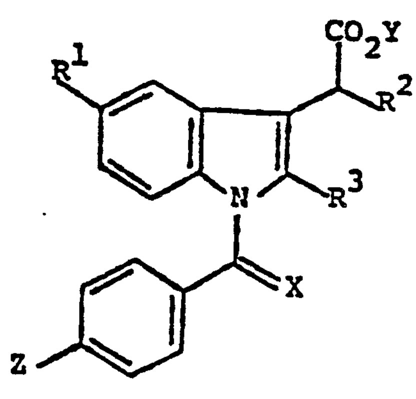

wherein $R^1$ is $-CH_3$, $-C_2H_5$, $-O-CH_3$ or -F, $R^2$ and $R^3$ are -H or $-CH_3$, Y is -H, an alkali metal, or $C_1$-$C_{20}$ alkyl, alkenyl or aryl, Z is a halogen, $-CF_3$, or $-S-CH_3$; and X is = 0 or $-(H)_2$.

EXAMPLE III

| | Ingredient | Percent by Weight of Total Composition |
|---|---|---|
| 1. | Petrolatum | 49% |
| 2. | Lidocaine hydrochloride | 3% |
| 3. | Glyceryl monoleate | 1% |
| 4. | Water | 47% |

This composition is topically applied to a human in need of local anesthesia as needed. 10 milligrams of total composition is applied per square centimeter of skin of the site in need of anesthesia.

In the foregoing composition, the following pharmaceutical agents can be substituted for the lidocaine: procaine, dibucaine, bupivacaine, tetracaine, chloroprocaine, hexylcaine, mepivacaine, piperocaine, prilocaine, cyclomethylcaine, dimethisoquin, benzocaine, dyclonine and pramoxine.

EXAMPLE IV

| | Ingredient | Percent by Weight of Total Composition |
|---|---|---|
| 1. | Petrolatum | 48% |
| 2. | Erythromycin base | 2% |
| 3. | Glyceryl monooleate | 1% |
| 4. | Water | 49% |

The composition is topically applied to a human suffering from a bacterial infection or acne at the site of the infection. 10 (ten) milligrams of total composition is applied per square centimeter of the afflicted situs.

In the foregoing composition the following pharmaceutical agents can be substituted for the erythromycin base: Penicillin V, oxacillin, cephalothin, cefazolin, cephaloridine, cephalexin, tetracycline, chlortetracycline, oxytetracycline, doxycycline, chloramphenicol, neomycin, polymyxin B, and bacitracin.

EXAMPLE V

| | Ingredient | Percent by Weight of Total Composition |
|---|---|---|
| 1. | Petrolatum | 45% |
| 2. | Glycerylmonolinoleate | 2.0 |
| 3. | Lecithin | 0.25% |
| 4. | Indomethacin | 0.5% |
| 5. | Propylene glycol | 20.0% |
| 6. | Water | 32.25% |

This composition is applied as in Example II.

EXAMPLE VI

| | Ingredient | Percent by Weight of Total Composition |
|---|---|---|
| 1. | Petrolatum | 30% |
| 2. | Glycerylmonolinoleate | 2.0% |
| 3. | Indomethacin | 0.5% |
| 4. | 1,4-butanediol | 20% |
| 5. | Water | 47.5% |

This composition is applied as in Example II.

EXAMPLE VII

As stated, the compositions of the present invention may be combined with other skin conditioning compositions. The water-in-oil emulsions of this invention are combined with a second emulsion of the oil-in-water type as follows.

First, parts 1 A and B (water-in-oil emulsions of the present invention) are prepared as follows:

| Ingredient | Part 1 Water-in-oil Emulsion A | B |
|---|---|---|
| Glycerine | 35% | 40% |
| Water | 33.5% | 38.5% |
| Lecithin | 1.5% | 1.5% |
| Petrolatum | 28.5% | 18.5% |
| Monoglyceride | 1.5% | 1.5% |

The glycerine, water and lecithin are thoroughly mixed. The monoglycerine is mixed into the petrolatum. This lipid phase

is heated to about 70°C, and mixed with the aqueous phase under high shear. The entire composition is allowed to come to room temperature. The water-in-oil emulsions of Part 1, (A and B), are then combined with an oil-in-water emulsion of the type described in U.S. Patent Application Serial Number 306,807, Dixon et al., previously incorporated by reference, which has been prepared by standard emulsion preparing techniques. Using a ratio of water-in-oil:oil-in-water of 1:4.4 for Composition A and 1:5.1 for Composition B, these two emulsions are gently folded together by a low shear, scraped wall blending method utilizing counter-rotated, interdigitated blending blades, such as a Sunbeam Mixmaster. The final compositions are as follows.

| Ingredient | Composition A | Composition B |
|---|---|---|
| Water | 76.15% | 78.09% |
| Glycerine | 9.90 | 10.00 |
| Ethanol | 0.88 | 0.90 |
| Petrolatum | 5.27 | 3.03 |
| Lecithin | .28 | .25 |
| Monoglyceride[1] | .28 | .25 |
| Copolyol[2] | 1.32 | 1.35 |
| Cetyl Alcohol | 2.64 | 2.71 |
| POE 100 Monostearate | 0.22 | 0.22 |
| Isopropyl Palmitate | 0.88 | 0.94 |
| Dimethicone[3] | 0.44 | 0.47 |
| Stearic Acid | 0.22 | 0.22 |
| Amerlate WFA[4] | 0.22 | 0.22 |
| Emphos F27-85[5] | 0.22 | 0.22 |
| Cyclomethicone[6] | 0.16 | 0.26 |
| Carbopol 934[7] | 0.13 | 0.14 |
| Tektamer 38[8] | 0.02 | 0.02 |
| NaOH | 0.17 | 0.18 |
| Perfume | 0.17 | 0.18 |
| $TiO_2$ | 0.17 | 0.18 |
| EDTA | 0.09 | 0.09 |

1. Myverol 18-98, offered by Eastman Chemical Products, distilled monoglycerides of oleic and linoleic acid.

2. 90% cyclomethicone, 10% copolyol polymer Dow Corning X2-3225C - Cyclomethicone has 5 dimethyl siloxane groups and copolyol polymer has the formula

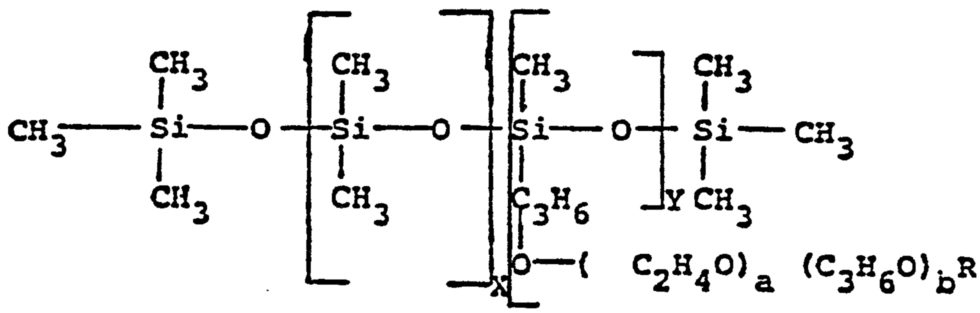

wherein x and y are selected such that the weight ratio of polydiorgano-siloxane segments to polyoxyalkylene segments is from about 2 to 8 and the mol ratio of a:(a+b) is from about 0.5 to 1.

3. Dow Corning 200 Fluid.

4. Lanolin acid offered by Amerchol

5. A glyceride phosphate ester offered by Witco Chemical Company.

6. Dow Corning X2-1201. A cyclic volatile silicone having five dimethyl siloxane groups.

7. A carboxy vinyl polymer offered by B. F. Goodrich Co.

8. 1,2-dibromo 2,4-dicyanobutane.

This composition provides effective skin conditioning when applied to dry skin and rubbed in. All percentages are by weight.

WHAT IS CLAIMED IS:

CLAIMS

1. An emulsion having a stable viscous isotropic dispersed phase for topical application, characterized in that it comprises:

(a) from 9% to 89% petrolatum;

(b) from 0.25% to 10% of an emulsifier selected from glyceryl monooleate, glyceryl monolinoleate and mixtures thereof;

(c) from 10% to 90% water; and

(d) from 0.01% to 25% of a skin conditioning agent.

2. A composition according to Claim 1, characterized in that the skin conditioning agent is selected from emollients, humectants, and mixtures thereof.

3. A composition according to Claim 1 or 2 characterized in that the skin conditioning agent is an emollient selected from glycerine, silicone oils, fatty alcohols, lanolin and its derivatives, polyhydric alcohols, phospholipids, polyhydric ethers, polyhydric alcohol esters, and mixtures thereof.

4. A composition according to Claim 1 or 2 characterized in that the skin conditioning agent is a humectant selected from sorbitol, propylene glycol, alkoxylated glucose, hexanetriol, and mixtures thereof.

5. A composition according to any of Claims 1-3 characterized in that the skin conditioning agent is glycerine and is present at a level of from 5% to 20%.

6. A composition according to any of Claims 1-5 characterized in that the emulsifier is present at a level of from 0.5% to 5%.

7. An emulsion having a stable viscous isotropic dispersed phase for topical application, characterized in that it comprises:

(a) from 10% to 50% petrolatum;

(b) from 0.5% to 5% of an emulsifier selected from glyceryl monooleate, glyceryl monolinoleate, and mixtures thereof;

(c) from 30% to 80% water; and

(d) from 5% to 20% glycerine.

8. A composition for skin conditioning, characterized in that it comprises a first emulsion according to any of Claims 1-7 having gently blended therein a second skin conditioning emulsion, said second emulsion being blended in said first emulsion in a weight ratio from from 1:99 to 99:1.

9. A composition according to Claim 8 characterized in that it contains a total level of unsaturated monoglyceride emulsifier of from 0.025 to 10%.

10. A method of conditioning skin characterized in that it comprises topically applying to skin in need of said conditioning a composition according to any of Claims 1-9.

0103910

European Patent Office

# EUROPEAN SEARCH REPORT

Application number

EP 83 20 0946

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X Y | US-A-3 957 969 (Y. FUJIYAMA) * Column 1, line 54 - column 2, line 31; claims 1,3,5,7,9,11,13 * | 1-10 | A 61 K 7/48 A 61 K 9/10 |
| D,Y | US-A-3 536 816 (H.J. KELLNER) * Column 2, lines 6-38; example 15; claims * | 2-6,8-10 | |
| D,Y | US-A-3 919 430 (F.P. SIEGEL) * Column 2, line 43 - column 3, line 20; claims * | 1-10 | |
| Y | SOAP, PERFUMERY & COSMETICS, vol. 54, no. 11, November 1981, pages 607-613, London, GB V. KINGLAKE: "The need for specialised formulae for the particular qualities of a baby's skin" * Page 613, 1st column, 2nd half, "Waterproof baby lotion" * | 1-10 | TECHNICAL FIELDS SEARCHED (Int. Cl. 3) A 61 K 7/00 A 61 K 9/00 |
| Y | FR-A-2 173 296 (UNILEVER N.V.) * Page 3, lines 5-14; example 1; claims 2,3 * | 1,6,7,9 | |

-/-

The present search report has been drawn up for all claims

| Place of search | Date of completion of the search | Examiner |
|---|---|---|
| THE HAGUE | 12-12-1983 | BERTE M.J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82

0103910

European Patent Office

# EUROPEAN SEARCH REPORT

Application number

EP 83 20 0946

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl. 3) |
|---|---|---|---|
| P,Y | CHEMICAL ABSTRACTS, vol. 97, no. 10, September 1982, page 423, no. 78917b, Columbus, Ohio, US & JP - A - 82 93 906 (LION CORP.) 11-06-1982 * Abstract * ----- | 1 | |

TECHNICAL FIELDS SEARCHED (Int. Cl. 3)

The present search report has been drawn up for all claims

| Place of search | Date of completion of the search | Examiner |
|---|---|---|
| THE HAGUE | 12-12-1983 | BERTE M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82